(19) Europäisches Patentamt
European Patent Office
Office européen des brevets

(11) Publication number: **0 189 142 A2**

(12) **EUROPEAN PATENT APPLICATION**

(21) Application number: 86100592.4

(22) Date of filing: 17.01.86

(51) Int. Cl.⁴: **C 07 C 43/23**, C 07 C 97/03, C 07 C 49/255, C 07 C 47/277, C 07 C 49/577, A 61 K 31/045, A 61 K 31/135, A 61 K 31/11, A 61 K 31/12

(30) Priority: 17.01.85 US 692132

(43) Date of publication of application: 30.07.86 Bulletin 86/31

(84) Designated Contracting States: AT BE CH DE FR GB IT LI LU NL SE

(71) Applicant: USV PHARMACEUTICAL CORPORATION, 1 Scarsdale Road, Tuckahoe New York (US)

(72) Inventor: Daw-Yuan Lee, Thomas, 54 Vernon Drive, Scarsdale New York (US)

(74) Representative: Patentanwälte Grünecker, Kinkeldey, Stockmair & Partner, Maximilianstrasse 58, D-8000 München 22 (DE)

# (54) Arylalkoxyphenyl-allylic alcohols as agents for the treatment of hypersensitive ailments.

(57) The present invention is concerned with the therapeutic composition comprising as an active ingredient a compound of the formula:

$$[(R_1)_{n'}\text{-}Ar_1\text{-}(X)_b\text{-}]_a[Ar\text{-}(CR=CR)_n\text{-}Z(R_2)_{n''}\text{-}(R_3)_{n'''}]_b \qquad I$$

and salts thereof,
wherein a = 1, 2 or 3; each b = 1 or 2 and is the same value as the other;
Ar is phenyl or naphthyl containing one or two substituents selected from the group consisting of hydrogen, alkoxy, aryloxy, hydroxy, hydroxyalkyl, alkyl, aryl, halo, $CF_3$, carboxy, carbalkoxy, carboxyalkoxy, carbalkoxyalkoxy, lower acyl, nitrilo, amino, nitro, mercapto or alkylthio;
$Ar_1$ is phenyl, naphthyl, or a nitrogen, oxygen or sulfur heterocyclic ring;
each $R_1$ is independently hydrogen, alkyl, carboxy, carbalkoxy, alkylenecarboxy, arylenecarboxy, alkylenecarbalkoxy, alkanoyl, formyl, nitrilo, amino, aminoalkylene, alkylamino, carboxamide, halo, trihalomethyl, hydroxy, alkoxy, aralkyloxy, aryloxy, nitro, sulfamyl, mercapto or alkylthio;
each R is H or alkyl;
Z is an alkylene chain containing up to 10 carbon atoms in the principal chain and up to a total of 12 carbon atoms and from 0 to 2 double bonds and Z when taken together with the carbon atom of (CR = CR) to which it is attached forms a cycloalkylidene ring;
each $R_2$ is a substituent attached to one of the carbon atoms of Z selected from the group consisting of $OR_4$, $SR_4$, $N(R_4)_2$, CHO and $COR_5$;
each $R_3$ is H, cycloalkyl, aralkyl, aryl, lower dialkylamino, morpholino, piperazino, piperidino or $CF_3$;
each $R_4$ is H, alkyl, benzoyl, lower alkanoyl, aryl or aralkyl;
$R_5$ is $OR_6$ or $N(R_4)_2$;
$R_6$ is H, alkyl, aryl, or aralkyl;
n = 1 or 2;
n' = 1 or 2;
n'' = 1 or 2;
n''' = 1 or 2;
X = $-O(CHR_7)_m-$, $-S(CHR_7)_m-$, $-NR_4(CHR_7)_m-$,
                         ||
                       $(O)_{m'}$
alkylene of up to 2 carbon atoms in the principal chain and up to a total of 4 carbon atoms, $-C(R_7) = C(R_7)-$, $-C\equiv C-$,
$-C-(CHR_7)_m-$, $-CH(CHR_7)_m-$, $-CH=N-$, $-CO-$, $-CS-$, $-CN(R_7)-$;
 ||                    |                                  ||      ||      ||
 O                   OH                                O      O      O
m = 0 or 1;
m' = 0, 1, or 2; and

EP 0 189 142 A2

$R_7$ is H, $CH_3$ or phenyl;
wherein the group(s) designated $[(R_1)_{n'}-Ar_1-(X)_b]_a$ is (are) attached directly to Ar.

In addition, the present invention relates to the method of using these compounds as lipoxygenase inhibitors possessing anti-inflammatory and anti-allergic responses.

0189142

## ARYLALKOXYPHENYL-ALLYLIC ALCOHOLS AS AGENTS FOR THE TREATMENT OF HYPERSENSITIVE AILMENTS

This invention relates to the use of certain chemical compounds possessing valuable pharmaceutical activity, particularly as lipoxygenase inhibitors possessing anti-inflammatory and antiallergic properties. Novel intermediates, used for preparing the active therapeutic agents, also possess valuable therapeutic properties, and are also within the scope of this invention.

Unsaturated carbonyl compounds are known in the prior art. EPO Appln. No. 71,399 describes the synthesis of compounds of the formula

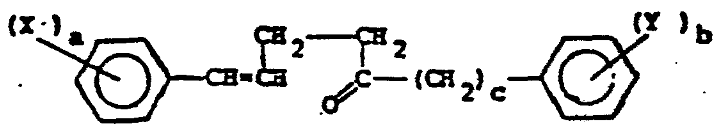

wherein X and Y are independently selected from -F, -OR, $S(O)_dR_1$, -CONHR, -NHR, $-CH_2OR$, -CN, -NHCONHR, -NHCSNHR, $-NHCOR^1$, $-SO_2NHR$, $-NHSO_2R^1$, and $-NHCO_2R$, wherein R is either hydrogen or lower alkyl moiety which is either a straight or branched hydrocarbon chain of 1-4 carbon atoms, $R^1$ is lower alkyl as defined above, or two Xs or two Ys taken together are $OCH_2O$-;

a, b, d is 0, 1 or 2;

c is 0, 1, 2 or 3;

These compounds are used as intermediates in the formation of pyrrolidines which are useful as regulators of the cardiovascular system and are also bronchodilators. No therapeutic properties of the carbonyl compounds are suggested or disclosed in this reference.

Various enones having the following formula

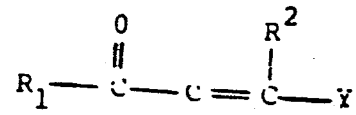

wherein $R_1$, $R_2$ = Me,Ph;
Y = OMe, O-tol-p, SEt, SPh, NHPr,

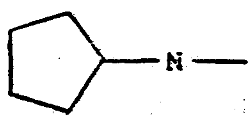 , NHPh.

were prepared by Kashima et al. as described in J. Heterocyclic Chem, 19, 1325-1328 (1982).

Ger. Offen. 3,011,258 describes the preparation of α,β unsaturated ketones of the formula

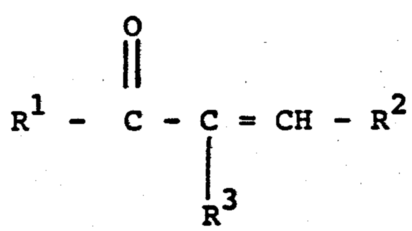

in which $R_1$ and $R_2$ are $C_1$-$C_6$ alkyl, napthyl or phenyl, with the phenyl moiety being substituted with F, Cl, Br, $C_1$-$C_4$ alkyl, and $C_1$-$C_4$ lower alkoxy, and $R_3$ is $C_1$-$C_4$ lower alkyl, benzyl or phenyl, with the benzyl and phenyl moieties being substituted with F, Cl, Br, or $C_1$-$C_4$ alkyl. These compounds are used as intermediates in the formation of triazolyl derivatives having fungicidal properties.

The preparation of 2,6-dibenzyloxybenzalacetone

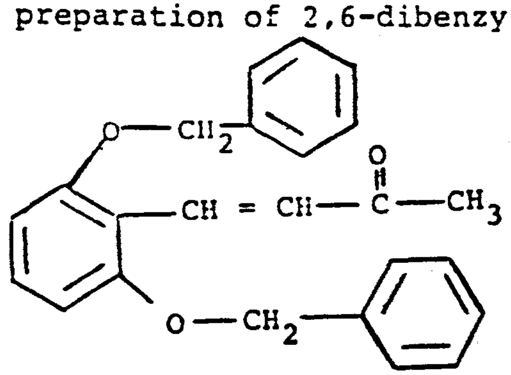

is described in Nippon Nogerkagaku Kashi, 34, 678-831 (1960); C.A. 59, 13929c (1963).

The preparation of 1-(3,4 dibenzyloxyphenyl)-4,4 dimethylpenten-3-one

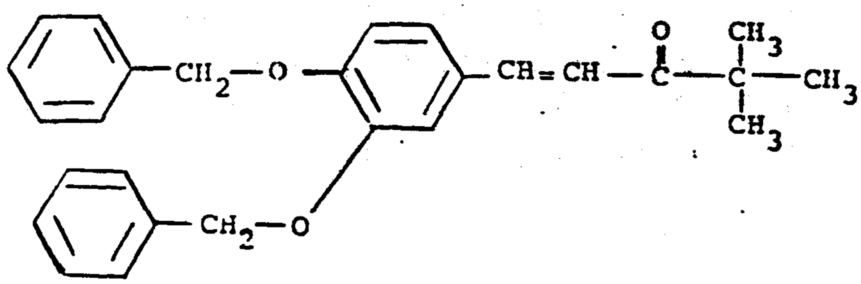

and its use as an antiviral agent is described in U.S. Patent No. 4,372,976.

Allylic alcohols are also known in the art. A compound having the formula

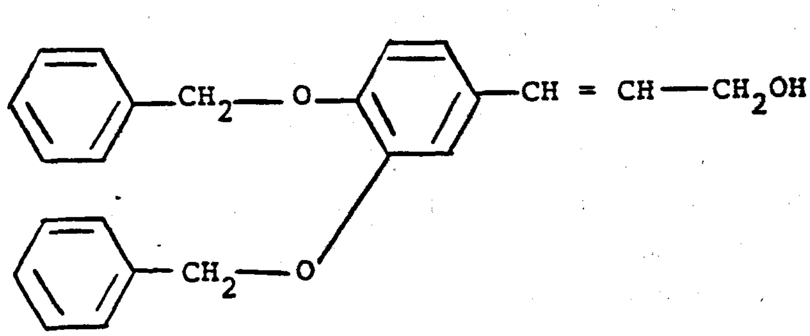

is described in EPO Application 71,399 (1983). This compound

is used as an intermediate in the formation of pyrrolidones which are useful as regulators of the cardiovascular system and are bronchodilators. No therapeutic activity of the alcohol was suggested or disclosed in this reference.

But, the therapeutic activity of these compounds as an antihypersensitive, an anti-inflammatory or as an anti-allergic agent was not recognized or suggested by these researchers. In addition, the conjugated diene analogs of the above compounds were not suggested or contemplated by these researchers.

Summary of the Invention

The present invention is concerned with the therapeutic composition comprising as an active ingredient a compound of the formula:

$$[(R_1)_{n'}-Ar_1-(X)_b-]_a[Ar-(CR=CR)_n-Z(R_2)_{n''}-(R_3)_{n'''}]_b \qquad \text{I}$$

and salts thereof,

wherein a = 1, 2 or 3; each b = 1 or 2 and is the same value as the other;

Ar is phenyl or naphthyl containing one or two substituents selected from the group consisting of hydrogen, alkoxy, aryloxy, hydroxy, hydroxyalkyl, alkyl, aryl, halo, $CF_3$, carboxy, carbalkoxy, carboxyalkoxy, carbalkoxyalkoxy, lower acyl, nitrilo, amino, nitro, mercapto or alkylthio;

$Ar_1$ is phenyl, naphthyl, or a nitrogen, oxygen or sulfur heterocyclic ring;

each $R_1$ is independently hydrogen, alkyl, carboxy, carbalkoxy, alkylenecarboxy, arylenecarboxy, alkylenecarbalkoxy, alkanoyl, formyl, nitrilo, amino,

aminoalkylene, alkylamino, carboxamide, halo, trihalomethyl, hydroxy, alkoxy, aralkyloxy, aryloxy, nitro, sulfamyl, mercapto or alkylthio;

each R is H or alkyl;

Z is an alkylene chain containing up to 10 carbon atoms in the principal chain and up to a total of 12 carbon atoms and from 0 to 2 double bonds and Z when taken together with the carbon atom of (CR=CR) to which it is attached forms a cycloalkylidene ring;

each $R_2$ is a substituent attached to one of the carbon atoms of Z selected from the group consisting of $OR_4$, $SR_4$, $N(R_4)_2$, CHO and $COR_5$;

each $R_3$ is H, cycloalkyl, aralkyl, aryl, lower dialkylamino, morpholino, piperazino, piperidino or $CF_3$;

each $R_4$ is H, alkyl, benzoyl, lower alkanoyl, aryl or aralkyl;

$R_5$ is $OR_6$ or $N(R_4)_2$;

$R_6$ is H, alkyl, aryl, or aralkyl;

n = 1 or 2;

n' = 1 or 2;

n" = 1 or 2;

n"' = 1 or 2;

X = $-O(CHR_7)_m-$, $-\underset{(O)_{m'}}{\underset{\|}{S}}(CHR_7)_m-$, $-NR_4\ (CHR_7)_m-$,

alkylene of up to 2 carbon atoms in the principal chain and up to a total of 4 carbon atoms, $-C(R_7) = C(R_7)-$, $-C\equiv C-$, $-\underset{O}{\underset{\|}{C}}-(CHR_7)_m-$, $-\underset{OH}{\underset{|}{C}}H(CHR_7)_m-$, $-CH=N-$, $-\underset{O}{\underset{\|}{C}}O-$, $-\underset{O}{\underset{\|}{C}}S-$,

$-\underset{O}{\underset{\|}{C}}N(R_7)-$;

m = 0 or 1;

m' = 0, 1, or 2; and

$R_7$ is H, $CH_3$ or phenyl;
wherein the group(s) designated $[(R_1)_{n'}-Ar_1-(X)_b]_a$ is (are) attached directly to Ar.

In addition, the present invention relates to the method of using these compounds as lipoxygenase inhibitors possessing anti-inflammatory and anti-allergic responses. Also encompassed within the invention are particularly useful intermediates in the preparation of compounds of Formula I, wherein $R_2$ is OH. These intermediates are composed of Formula II:

$$[(R_1)_{n'}-Ar_1-(X)_b]_a-Ar-(CR=CR)_n-Z_1-\underset{\displaystyle O}{\overset{\|}{C}}R']_b$$

II

wherein Ar, $Ar_1$, X, R, $R_1$, n, n', a and b are as defined above;

$Z_1$ is an alkylene chain containing from 0-10 carbon atoms in the principal chain and up to a total of 12 carbon atoms and from 0 to 2 double bonds;

$R_1$ is H, alkyl, cycloalkyl, aralkyl, aryl, or dialkylaminoalkyl;

wherein the group(s) designated $[(R_1)_{n'}\ Ar_1-(X)_b]_a$ is (are) attached directly to Ar.

The heterocyclic rings exemplary of Ar and $Ar_1$ contain at least one oxygen, sulfur or nitrogen and include the so-called benzoheterocyclic rings. Exemplary heterocyclics include furan, thiophene, pyrrole, pyridine, thiazole, piperazine, oxazole, benzofuran, quinoline, indole, benzothiophene, benzoxazole and similar heterocyclic rings as well as the N-oxides of the nitrogen-heterocyclics. The preferred heterocyclics are quinoline and indole.

Compounds which are particularly preferred are those in which Ar is phenyl or naphthyl, and most preferred are those in which Ar and $Ar_1$ are each phenyl.

The alkyl groups, either alone or within the various substituents defined hereinbefore are preferably lower alkyl which may be straight or branched-chain, and include methyl, ethyl, propyl, isopropyl, butyl, isobutyl, tert-butyl, amyl, hexyl and the like.

The halo atoms in halo and trihalomethyl are Cl, Br, I and preferably F. The aryl groups are preferably phenyl or naphthyl.

The preferred compounds of Formula I are those in which $R_2$ is $OR_4$, n is 1 or 2, n" is 1 or 2 and Z contains up to five carbon atoms in the principal chain and is attached directly to $(CR{=}CR)_n$ by a covalent bond. The preferred compounds of Formula II are those in which n is 1 or 2, n" is 1 and Z contains 0 to 6 carbon atoms.

The aryl groups, i.e., phenyl, the aryl group of arylalkyl, and benzoyl, may be unsubstituted or substituted with one or two substituents such as OH, alkoxy, aryloxy, arylalkyloxy, halogen, alkyl, carboxy, carbalkoxy, carboxamide and similar such groups.

The present compounds can be prepared by art-recognized procedures from known compounds or readily-preparable intermediates. An exemplary general procedure is as follows:

$$\underset{\text{III}}{(R_1)_{n'}\text{-}Ar_1\text{-}X\text{-}Ar\text{-}CHO} + \underset{\text{IV}}{\overset{R}{\overset{|}{C}}H_2\text{-}(CR{=}CR)_{n-1}\text{-}Z(R_2)_{n''}\text{-}(R_3)_{n'''}} \longrightarrow \text{I}$$

Alternatively, the compounds of Formula III may include one (CR=CR) group between Ar and the aldehyde group and the (CR=CR) group eliminated from compounds of formulae IV and V. In this alternate procedure, there are produced compounds in which n=2.

Compounds in which $R_2$ is OH can be directly prepared by reduction of the ketones of Formula II formed from the following reaction:

$$\text{III} + \underset{\text{V}}{\overset{R}{\overset{|}{C}H_2}-(CR{=}CR)_{n-1}-Z_1-\underset{O}{\underset{\|}{C}}-R'} \longrightarrow \text{II} \longrightarrow \text{I}$$

Alternatively, the reaction of a Grignard reagent with an aldehyde intermediate of suitable carbon content can directly form the corresponding secondary alcohol:

$$\underset{\text{VI}}{[(R_1)_n-Ar_1(X)_b]_a-Ar-(CR{=}CR)_n-CHO} \quad +$$

$$\underset{\text{VII}}{MgX\ Z_2(R_2)_{n''-1}(R_3)_{n'''}} \longrightarrow \text{the corresponding } 2° \text{ alcohol.}$$

In this reaction, $Z_2$ is one carbon less than Z. Of course, the aldehyde group in structure VI may be separated by an alkylene chain from the (CR=CR) group so that the secondary alcohol can be placed as desired in the alkylene chain representative of Z.

Various preparative procedures are illustrated in the specific examples which are included herein.

The present compounds form salts with acids when a basic amino function is present and salts with bases when an acid function, i.e., carboxyl, is present. All such salts

are useful in the isolation and/or purification of the new products. Of particular value are the pharmaceutically acceptable salts with both acids and bases. Suitable acids include, for example, hydrochloric, sulfuric, nitric, benzenesulfonic, toluenesulfonic, acetic, malic, tartaric and the like which are pharmaceutically acceptable. Basic salts for pharmaceutical use are the Na, K, Ca and Mg salts.

The compounds of the present invention can be administered to the host in a variety of forms adapted to the chosen route of administration, i.e., orally, intravenously, intramuscularly or subcutaneous, topically or inhalation routes.

The active compound may be orally administered, for example, with an inert diluent or with an assimilable edible carrier, or it may be enclosed in hard or soft shell gelatin capsule, or it may be compressed into tablets, or it may be incorporated directly with the food of the diet. For oral therapeutic administration, the active compound may be incorporated with excipients and used in the form of ingestible tablets, buccal tablets, troches, capsules, elixirs, suspensions, syrups, wafers, and the like. Such compositions and preparations should contain at least 0.1% of active compound. The percentage of the compositions and preparations may, of course, be varied and may conveniently be between about 2 to about 60% of the weight of the unit. The amount of active compound in such therapeutically useful compositions is such that a suitable dosage will be obtained. Preferred compositions or preparations according to the present invention are prepared so that an oral dosage unit form contains between about 50 and 300 mg of active compound.

The tablets, troches, pills, capsules and the like may also contain the following: A binder such as gum

tragacanth, acacia, corn starch or gelatin; excipients such as dicalcium phosphate; a disintegrating agent such as corn starch, potato starch, alginic acid and the like; a lubricant such as magnesium stearate; and a sweetening agent such as sucrose, lactose or saccharin may be added or a flavoring agent such as peppermint, oil of wintergreen, or cherry flavoring. When the dosage unit form is a capsule, it may contain, in addition to materials of the above type, a liquid carrier. Various other materials may be present as coatings or to otherwise modify the physical form of the dosage unit. For instance, tablets, pills, or capsules may be coated with shellac, sugar or both. A syrup or elixir may contain the active compound, sucrose as a sweetening agent, methyl and propylparabens as preservatives, a dye and flavoring such as cherry or orange flavor. Of course, any material used in preparing any dosage unit form should be pharmaceutically pure and substantially non-toxic in the amounts employed. In addition, the active compound may be incorporated into sustained-release preparations and formulations.

The active compound may also be administered parenterally or intraperitoneally. Solutions of the active compound as a free base or pharmacologically acceptable salt can be prepared in water suitably mixed with a surfactant such as hydroxypropylcellulose. Dispersions can also be prepared in glycerol, liquid polyethylene glycols, and mixtures thereof and in oils. Under ordinary conditions of storage and use, these preparations contain a preservative to prevent the growth of microorganisms.

The pharmaceutical forms suitable for injectable use include sterile aqueous solutions or dispersions and sterile powders for the extemporaneous preparation of sterile injectable solutions or dispersions. In all cases the form

must be sterile and must be fluid to the extent that easy syringability exists. It must be stable under the conditions of manufacture and storage and must be preserved against the contaminating action of microorganisms such as bacteria and fungi. The carrier can be a solvent or dispersion medium containing, for example, water, ethanol, polyol (for example, glycerol, propylene glycol, and liquid polyethylene glycol, and the like), suitable mixtures thereof, and vegetable oils. The proper fluidity can be maintained, for example, by the use of a coating such as lecithin, by the maintenance of the required particle size in the case of dispersion and by the use of surfactants. The prevention of the action of microorganisms can be brought about by various antibacterial and antifungal agents, for example, parabens, chlorobutanol, phenol, sorbic acid, thimerosal, and the like. In many cases, it will be preferable to include isotonic agents, for example, sugars or sodium chloride. Prolonged absorption of the injectable compositions can be brought about by the use in the compositions of agents delaying absorption, for example, aluminum monostearate and gelatin.

Sterile injectable solutions are prepared by incorporating the active compound in the required amount in the appropriate solvent with various of the other ingredients enumerated above, as required, followed by filtered sterilization. Generally, dispersions are prepared by incorporating the various sterilized active ingredients into a sterile vehicle which contains the basic dispersion medium and the required other ingredients from those enumerated above. In the case of sterile powders for the preparation of sterile injectable solutions, the preferred methods of

preparation are vacuum drying and the freeze-drying technique which yield a powder of the active ingredient plus any additional desired ingredient from previously sterile-filtered solution thereof.

The following examples further illustrate the invention.

EXAMPLE 1

4-(p-Benzyloxyphenyl)-3-buten-2-one(1)

A mixture of p-benzyloxybenzaldehyde (40g), acetone (100 ml), ethanol (300 ml) and 2N aqueous NaOH solution (1 ml) was stirred at room temperature overnight. The precipitate was collected and recrystallized with acetone to give a crystalline product in quantitative yield, m.p. 98-101°C. (lit. m.p. 106-107°C.).

Similarly prepared, using appropriate substituted benzaldehyde and ketone as starting material, were compounds 2, 4-11, 20, and 43.

EXAMPLE 2

8-(p-Benzyloxyphenyl)-2,2-dimethyl-5,7-octadien-4-one(13)

A mixture of p-benzyloxycinnamaldehyde (3 g), 4,4-dimethyl-pentan-2-one (0.85 g), ethanol (15 ml) and 60% aqueous KOH solution (24 ml) was stirred at room temperature overnight. After dilution with water followed by neutralization with hydrochloric acid, a solid precipitated out which was collected, washed with water and recrystallized with ethanol to yield a crystalline product (1.9 g), m.p. 88-89°C.

Similarly prepared were compounds 14-16.

EXAMPLE 3

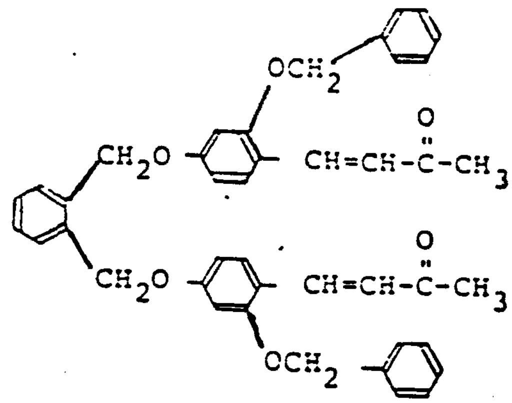

This Bis-ketone (18)

A mixture of 2,4-dihydroxybenzaldehyde (13.6 g), ortho-2,2'-dibromoxylene (16 g), anhydrous potassium carbonate (40 g) in DMF (200 ml) was stirred in an oil bath at 140°C for one hour. After removal of solvent in reduced pressure, the organic layer was dried by anhydrous magnesium sulfate and evaporated to give an oil residue. Purification with a silica gel dry column (chloroform as the eluant) afforded the bis-hydroxybenzaldehyde intermediate as a white solid (3.8 g).

A mixture of the above solid (3.8 g), benzyl chloride (15 ml), anhydrous potassium carbonate (23 g) in DMF (100 ml) was stirred in an oil bath at 140°C for one hour. It was then worked up in a manner similar to above and the bis-benzyloxybenzalaldehyde precursor was obtained as a white solid (2.7 g), m.p. 102-104°C.

A mixture of this precursor (2.7 g) in acetone (30 ml), ethanol (30 ml) and 2N NaOH solution (1 ml) was refluxed for one hour. After cooling, the precipitate was collected

and recrystallized with chloroform-ether to yield a crystalline product (1.6 g), m.p. 157-159 °C.

EXAMPLE 4

4-(p-Diphenylmethoxyphenyl)-3-buten-2-one (19)

A mixture of chlorodiphenylmethane (10.0 g), 4-hydroxybenzaldehyde (6.1 g), anhydrous potassium carbonate (6.9 g) in DMF (50 ml) was stirred in an oil bath at 140°C for one hour. The mixture was filtered, then evaporated to a residue which was partitioned between dichloromethane and water. The organic layer was dired with anhydrous magnesium sulfate then evaporated to give the crude p-diphenylmethoxybenzaldehyde as a white solid (3.2 g).

A mixture of p-diphenylmethoxybenzaldehyde (3.2 g), acetone (5.5 ml), ethanol (5.5 ml) and 2N NaOH solution (0.66 ml) was stirred at room temperature for one hour. The mixture was then evaporated to give an oily residue which was put through a silica gel dry column (ethyl acetate: hexane = 2:1 as the eluant). The pure product was obtained first as an oil but solidified after standing at room temperature overnight. Filtration with hexane yielded a yellowish solid product (1.5 g), m.p. 97°C.

EXAMPLE 5

1-(2',4'-Dibenzyloxyphenyl)-1-octen-3-one (3)

A mixture of 2,4-dibenzyloxybenzaldehyde (5.7 g), 2-oxo-heptyltriphenylphosphorane (14 g) in toluene (50 ml) was stirred and heated around 110°C for three hours. After cooling to room temperature, ethyl acetate (200 ml) was added. The organic layer was washed in turn with 1N HCL solution, water, and aqueous sodium bicarbonate solution, dried by anhydrous magnesium sulfate and evaporated. The residue was purified via a silica gel dry column (10% ethyl acetate in hexane as the eluant) followed by trituration with ether-hexane to afford the product as a slightly sticky solid (4.5 g), m.p. 60-70°C.

Similarly prepared, using 4-benzyloxycinnamaldehyde as the starting material, compound 8 was obtained, m.p. 117-118°C.

EXAMPLE 6

1-(p-Benzyloxyphenyl)-5-N,N-dimethylamino-1-penten-3-one (17)

A mixture of 4-p-benzyloxyphenyl-3-buten-2-one (1, 100 g), paraformaldehyde (72.8 g), dimethylamine hydrochloride (36.4 g) in ethanol (300 ml) and 2N HCL solution (2 ml) was stirred and refluxed for one hour. The reaction mixture was concentrated at reduced pressure to a small volume and then partitioned between 2N HCL solution and ethyl acetate. The acidic solution was basified with 50% NaOH solution to give out a red colored oil which upon trituration with ether became a wet solid. Recrystallization with chloroform-hexane afforded the product as a crystalline solid (42 g), m.p. 73-80°C.

EXAMPLE 7

4-(p-Benzyloxyphenyl)-3-buten-2-ol (21)

To a solution of 4-(p-benzyloxyphenyl)-3-buten-2-one ( 1, 4.5 g ) in a mixture of tetrahydrofuran (50 ml) and ethanol (250 ml) was added sodium borohydride (5.3 g) with stirring at cold temperature. The reaction mixture was stirred for one hour, then quenched with 10% aqueous solution of ammonium chloride. After concentration at reduced pressure, it was extracted with chloroform. The organic layer was dried by anhydrous magnesium sulfate followed by evaporation to give a solid residue (4 g) which was recrystallized from ethyl acetate-ethyl ether-hexane to afford the product as a white crystalline solid, m.p. 111-112°C.

Similarly prepared were alcohols 22-40, 42, and 43.

In the following tables, the compounds are denoted by numbers. These numbers correspond to the compound identification number, which are located in parentheses after the name in the heading in the preceding examples.

TABLE 1

INTERMEDIATES

$[(R_1)_{n'}-Ar_1-(X)_b]_a-[Ar-(CR=CR)_n-Z_1[\overset{O}{\overset{\|}{C}}-R']_b$

| $(R_1)_{n'}Ar_1$ | X | b | a | Ar | R | n | $Z_1\underset{\underset{O}{\|}}{C}-R'$ | M.Pt. (0°C) |
|---|---|---|---|---|---|---|---|---|
| 1. phenyl | $CH_2O$ | 1 | 1(4-) | phenyl | H | 1 | $-COCH_3$ | 98-101* |
| 2. phenyl | $CH_2O$ | 1 | 2(2,4-) | phenyl | H | 1 | $-COCH_3$ | 86-92 |
| 3. phenyl | $CH_2O$ | 1 | 2(2,4-) | phenyl | H | 1 | $-CO(n-C_5H_{11})$ | 60-70 |
| 4. phenyl | $CH_2O$ | 1 | 2(2,4-) | phenyl | H | 1 | $COCH_2$(3°butyl) | 92-94 |
| 5. phenyl | $CH_2O$ | 1 | 2(2,4-) | phenyl | H | 1 | CO(3°butyl) | 84-86 |
| 6. 4'F-phenyl | $CH_2O$ | 1 | 2(2,4-) | phenyl | H | 1 | $-COCH_3$ | 139-140 |
| 7. phenyl | $CH_2O$ | 1 | 2(2,5-) | phenyl | H | 1 | $-COCH_3$ | 118-121 |
| 8. phenyl | $CH_2O$ | 1 | 2(3,4-) | phenyl | H | 1 | $COCH_3$ | 101-103 |
| 9. phenyl | $CH_2O$ | 1 | 2(3,4-) | phenyl | H | 1 | CO(3°butyl) | 78-80 |
| 10. phenyl | $CH_2O$ | 1 | 3(2,3,4-) | phenyl | H | 1 | $-COCH_3$ | 89-91 |
| 11. phenyl | $CH_2O$ | 1 | 3(2,4,6-) | phenyl | H | 1 | $-COCH_3$ | 94-96 |
| 12. phenyl | $CH_2O$ | 1 | 1(4-) | phenyl | H | 2 | $CO(n-C_5H_{11})$ | 117-118 |
| 13. phenyl | $CH_2O$ | 1 | 1(4-) | phenyl | H | 2 | $COCH_2$(3°butyl) | 88-89 |
| 14. phenyl | $CH_2O$ | 1 | 1(4-) | phenyl | H | 2 | CO(3°butyl) | oil |

* lit mp is 106-107°C

TABLE 1 (cont.)

| | | | | | | | | |
|---|---|---|---|---|---|---|---|---|
| 15.phenyl | $CH_2O$ | 1 | 2(2,4-) | phenyl | H | 2 | $COCH_2$(3°butyl) | oil |
| 16.phenyl | $CH_2O$ | 1 | 2(2,4-) | phenyl | H | 2 | CO(3°butyl) | oil |
| 17.phenyl | $CH_2O$ | 1 | 1(4-) | phenyl | H | 1 | $CO(CH_2)_2N(CH_3)_2$ | 73-80 |
| 18.phenyl | $CH_2O$ | 2** | 1(4-) | phenyl | H | 1 | $COCH_3$ | 157-5 |
| 19.phenyl | ØCHO | 1 | 1(4-) | phenyl | H | 1 | $COCH_3$ | 97 |
| 20. 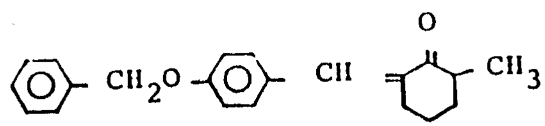 | | | | | | | | 80-85 |
| 41.phenyl | $CH_2O$ | 1 | 2(2,4-) | phenyl | H | 1 | -CHO | 79-84 |
| 43.phenyl | $CH_2O$ | 1 | 1(2-) | naphthalyl | H | 1 | $-COCH_3$ | 66-69 |

** ortho positioned on $Ar_1$.

The allylic alcohols 21-40, and 42 and 44 were prepared by mild reduction of the corresponding keto groups in compounds 1-20, 41, and 43 respectively, by the reaction of sodium borohydride as described. The melting points are given in tabular form as follows:

| Compounds | M.Pt. (°C.) |
|---|---|
| 21 | 111-112 |
| 22 | 68-70 |
| 23 | 70-73 |
| 24 | 79-80 |
| 25 | 76-78 |
| 26 | 71-72 |
| 27 | 93-96 |
| 28 | 68-71 |
| 29 | 38-43 |
| 30 | oil |
| 31 | 111-113 |
| 32 | 83-84 |
| 33 | 51-52 |
| 34 | 83-84 |
| 35 | oil |
| 36 | 50 |
| 37 | 202-204* |
| 38 | 110-113 |
| 39 | 46-47 |
| 40 | 96-98 |
| 42 | 101-102 |
| 44 | oil |

*: HCL salt

Using the foregoing procedures, compounds of formula I and II can be prepared in which $Ar_1$ is quinolyl, indolyl, benzothienyl, benzofuranyl and benzoxazolyl from corresponding starting compounds.

The compounds of the present invention have potent activity in regulating the formation of lipoxygenease and as such possess therapeutic value in the treatment of inflammatory conditions and allergic responses such as anaphlaxis and asthma.

Lipoxygenases in mammals have been found in the lung, platelets, and white cells. They are enzymes capable of oxidizing arachidonic acid into hydroperoxyeicosatetraenoic acids (HPETEs) and their stable products hydroxyeicosatetraenoic acids (HETEs). Lipoxygeneases are classified according to the position in the arachidonic acid which is oxygenated. Platelets metabolize arachidonic acid to 12-HETE, while polymorphonuclear leukocytes contain 5 and 15 lipoxygenases. It is known that 12-HETE and 5, 12-diHETE are chemotactic for human neutrophils and eosinophils, and may augment the inflammation process. 5-HPETE is known to be a precursor of slow-reacting substance of anaphylaxis (SRS-A). The SRS family of molecules, such as leukotrienes B, C, and D, have been shown to be potent bronchoconstrictors (see, NATURE 288, 484-486 (1980)).

The following protocol describes an assay to detect inhibitors of the lipoxygenase pathway. Such inhibitors are believed to be capable of modulating the biosynthesis of the leukotrienes, a property believed to be useful in treating asthma and inflammatory disease states.

<u>Protocol for Detecting Inhibitors of the Lipoxygenase Pathway</u>

A suspension of rat neutrophils in buffer is incubated for 3 minutes at 30°C with [$^{14}$C]-arachidonic acid (AA) and Calcium Ionophore A23187. Citric acid (2M) is used to quench the reaction. Following the addition of a trace amount of ($^{3}$H)-5-HETE together with an excess of unlabeled 5-HETE to each tube, the mixture is extracted with chloroform/methanol. The organic layer is washed with dilute acid and an aliquot is transferred to glass tubes and dried. The residue is dissolved in a small volume of chloroform and an aliquot is spotted on silica gel TLC sheets, which are developed with an ethyl acetate/isooctane/water/acetic acid solvent system. The 5-HETE spots are visualized with iodine, cut out and placed in scintillation vials for counting. After adjusting for the extraction efficiency, the amount (pmole) of [$^{14}$C]-5-HETE in each of the tubes is quantitated by substracting the net pmoles of 5-HETE in the tubes containing buffer alone (blank) from the pmoles of 5-HETE in the tubes containing buffer and cells (control). The ability of the test compounds to modulate the activity of this enzyme is determined by a decrease or increase in the net amount of 5-HETE produced.

Some compounds in this invention also display potent activities in regulating phospholipases and as such possess therapeutic value in the treatment of inflammatory conditions.

Inflammatory responses to a variety of offending stimuli are promoted by products of arachidonic acid metabolism. These products include leukotrienes (SRS-A), prostaglandins, prostacyclin and its metabolites, and thromboxanes. No matter what combination of products results from passage of substrate down the branches of this complex

cascade, the initial step involves the release of arachidonic acid from phospholipids or from triglycerides containing this long-chain fatty-acid [1]. The enzymes catalyzing such release of arachidonic acid are:
(a) phospholipase C followed by diglyceride lipase [2];
(b) phospholipase $A_2$, either soluble or membrane-bound[3,4]; and (c) a lipase able to degrade triglycerides that contain arachidonic acid[1].

---

[1] Borgeat, P., M. Hamberg, and B. Samuelson. Transformation of arachidonic acid and homo- -linolenic acid by rabbit polymorphonuclear leukocytes. J. Biol. Chem., 251: 7816-7810 (1976).

[2] Bell, R.L., D.A. Kennerly, N. Stanford, and P.W. Majerus. Diglyceridelipase: A pathway for arachidonate release from human platelets. Proc. Nat. Acad. Sci., U.S. 76: 3238-3241 (1979).

[3] Vadas, P., and J.B. Hay. The release of phospholipase $A_2$ from aggregated platelets and stimulated macrophages of sheep. Life Sciences, 26: 1721-1729 (1980).

Two assays have been developed to test the ability of the invented compounds on the activity of the phospholipases. In one protocol, a procedure is described for testing the inhibitory effects of these compounds on Phospholipase C (PLC), while the other protocol describes a means for testing the inhibitory effect of these compounds on Phospholipase $A_2$ ($PLA_2$).

<u>Protocol for In Vitro Assay for Inhibitors of Phospholipase</u>

The PLC employed in this screen is obtained by aggregation of purified rat platelets in the presence of $CaCl_2$ and ADP. In the enzyme assay, phosphatidylinositol having $^3H$-labeled arachidonate residues at R2 is employed as substrate. PLC acts by cleaving the phosphate ester bond yielding diglyceride as follows:

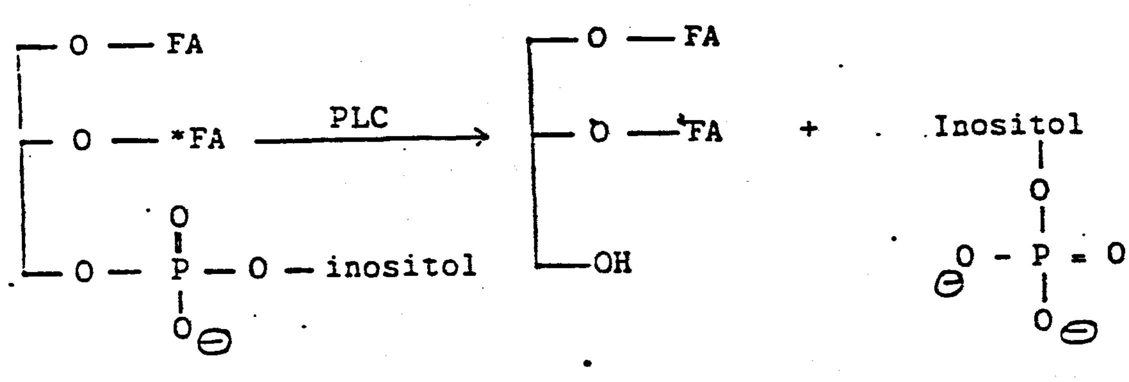


$^3H$-Phosphatidylinositol $^3H$-diglyceride Inositol

---

(cont.)

[4] Franson, R.C., D. Eisen, R. Jesse, and C. Lanni. Inhibition of highly purified mammalian phospholipases $A_2$ by non-steroidal anti-inflammatory agents, modulation by calcium ions. Biochemical J., 186: 633-636 (1980).

Following completion of the reaction, the assay medium is acidified and extracted with hexane which takes up unreacted substrate and diglyceride. The hexane extract is passed over a short silica gel column which retains 99% of the phosphatidylinositol. The $^3H$-labeled diglyceride is not retained (95% recovery in eluate) and is collected directly in scintillation counting vials. The diglyceride is conveniently quantitated by liquid scintillation spectrometry.

The compounds were tested at 300 uM in a buffer containing 0.06 mM unlabeled phosphatidylcholine (PC), 20-30,000 cpm of $^{14}C$ [PC], 150 mM NaCl, 5 mM $CaCl_2$ and 50 mM Tris(hydroxymethyl) methylaminopropanesulfonic acid buffer, adjusted to pH 9.0 with 1N NaOH. The temperature of the buffer is maintained at a temperature of 37°C. The reaction was initiated by addition of the enzyme and it was terminated 10 minutes later by addition of 1 ml of 1 N HCl.

Following acidification, the samples were extracted with 2 ml of isopropyl alcohol and 2 ml of hexane, vortexed and allowed to stand until the phrases separate. Free inositol and some unreacted substrate were taken up in the isopropanol-saturated hexane. The hexane phase of the extraction mixture was transferred to a short silica gel column which retained unreacted phosphatidylinositol, but not the $^3H$-diglyceride. The column effluent was collected directly in scintillation vials. The columns were washed once with additional 2 ml of hexane. The radiolabeled diglycerides were quantitated by liquid scintillation spectrometry.

Protocol for In Vitro Assay for Inhibitors of Phospholipase $A_2$ Assayed at pH 7.0 ($PLA_2$)

The $PLA_2$ employed in this screen is obtained by aggregation of purified rat platelets. In the enzyme assay phosphatidylcholine having $^{14}C$-labeled palmitate residues at R1 and R2 is employed as substrate. $PLA_2$ acts by cleaving the $R_2$ fatty acid ester bond yielding free fatty acid and lysophosphatidyl choline as follows:

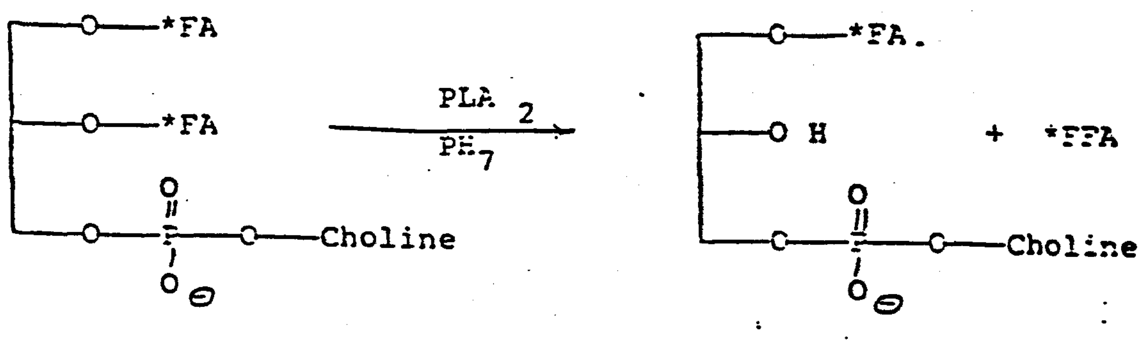


$^{14}C$-Phosphatidyl choline

$^{14}C$-Lysophosphatidyl choline

$^{14}C$-Palmitic acid

Following completion of the reaction, the assay medium is acidified and extracted with hexane, which takes up unreacted substrate and free fatty acid product. The hexane extract is passed over a short silica gel column which retains 99% of the phosphatidyl choline. The $^{14}C$-labeled palmitic acid is not retained (90% recovery in eluate) and is collected directly in scintillation counting vials. The released palmitic acid is conveniently quantitated by liquid scintillation spectrometry.

The compounds were tested at 100 uM in a buffer containing 0.3 mM unlabeled phosphatidylcholine (PC), 20-30,000 cpm of $^{14}C$ [PC], 100 mM NaCl, 1 mM $CaCl_2$ and 50 mM

Tris-HCL adjusted to pH 7.2 with 1 N NaOH. This resulted in a buffer at pH 7.2 The temperature of the buffer is maintained at a temperature of 37°C. The reaction was initiated by addition of the enzyme and it was terminated 30 minutes later by the addition of 100 ml of 1 N HCL.

Following acidification, the complex was extracted with 2 ml of 2-propanol and 2 ml of hexane, vortexed, and allowed to stand until the phases separated. Free fatty acids (FFA) and some unreacted substrate were taken up in the isopropanol-saturated hexane. The hexane phase of the extraction mixture was transferred to a short silica gel column which retained unreacted PC but not the FFA. The column effluent was collected directly in scintillation vials. The columns were washed once with an additional 2 ml of hexane. The radiolabeled FFA were quantitated by liquid scintillation spectrometry.

Table 3

Activities of Allylic Alcohols

| compound | Rat PMN 5-lipoxygenase inhibition ($I_{50}$) | ($PLA_2$) Phospholipase $A_2$ inhibition ($I_{50}$) | (PLC) Phospholipase C inhibition ($I_{50}$) |
|---|---|---|---|
| 21 | 1.5 uM | 120 uM | |
| 22 | 1 uM | 90 uM | |
| 23 | 10 uM | | |
| 24 | 0.3 uM | 100 uM | |
| 25 | 1.1 uM | 80 uM | |
| 28 | 2 uM | | |
| 29 | 2.3 uM | | |
| 32 | 7 uM | | |
| 34 | 4.5 uM | | |
| 37 | | | 24 uM |
| 39 | 3.5 uM | 31 uM | |
| 40 | | 170 uM | |
| 42 | 1.4 uM | | |

The results of these tests on allylic alcohols are tabulated in the above Table 3. In Table 3, the compounds are denoted by the numbers in parentheses after the name in the heading in the examples. As the data indicate, the allylic alcohols are good inhibitors of leukotrienes and phospholipases. As a result, these allylic alcohols have therapeutic value in the treatment of inflammatory conditions and allergic responses.

WHAT IS CLAIMED IS:

1. A therapeutic composition comprising as an active ingredient a compound of the formula:

$$[(R_1)_{n'}Ar_1-(X)_b-]_a[Ar-(CR=CR)_n-Z(R_2)_{n''}-(R_3)_{n'''}]b$$

and salts thereof,

wherein a = 1, 2 or 3; each b = 1 or 2 and is the same value as the other;

Ar is phenyl or naphthyl containing one or two substituents selected from the group consisting of hydrogen, alkoxy, aryloxy, hydroxy, hydroxyalkyl, alkyl, aryl, halo, $CF_3$, carboxy, carbalkoxy, carboxyalkoxy, carbalkoxyalkoxy, lower acyl, nitrilo, amino, nitro, mercapto, or alkylthio;

$Ar_1$ is phenyl, naphthyl, or a nitrogen, oxygen or sulfur heterocyclic ring;

each $R_1$ is independently hydrogen, alkyl, carboxy, carbalkoxy, alkylenecarboxy, arylenecarboxy, alkylenecarbalkoxy, alkanoyl, formyl, nitrilo, amino, aminoalkylene, alkylamino, carboxamide, halo, trihalomethyl, hydroxy, alkoxy, aralkyloxy, aryloxy, nitro, sulfamyl, mercapto or alkylthio;

each R is H or alkyl;

Z is an alkylene chain containing up to 10 carbon atoms in the principal chain and up to a total of 12 carbon atoms and from 0 to 2 double bonds and Z when taken together with the carbon atom of (CR=CR) to which it is attached forms a cycloalkylidene ring;

each $R_2$ is $OR_4$ attached to one of the carbon atoms of Z;

each $R_3$ is H, cycloalkyl, aralkyl or aryl, dialkylamino, morpholino, piperazino, piperidino, or $CF_3$;

each $R_4$ is H, alkyl, benzoyl, lower alkanoyl, aryl

or aralkyl;

$n = 1$ or 2;

$n' = 1$ or 2;

$n'' = 1$ or 2;

$n''' = 1$ or 2;

$X = -O(CHR_7)_m-$, $-\underset{\underset{(O)_{m'}}{\|}}{S}(CHR_7)_m-$, $-NR_4(CHR_7)_m-$,

alkylene of up to 2 carbon atoms in the principal chain and up to a total of 4 carbon atoms, $-C(R_7)=C(R_7)-$, $-C\equiv C-$, $-\underset{\underset{O}{\|}}{C}-(CHR_7)_m-$, $-\underset{\underset{OH}{|}}{C}H(CHR_7)_m-$, $-CH=N-$, $-\underset{\underset{O}{\|}}{C}O-$, $-\underset{\underset{O}{\|}}{C}S-$,

$-\underset{\underset{O}{\|}}{C}N(R_7)-$;

$m = 0$ or 1;

$m' = 0$, 1 or 2; and

$R_7$ is H, $CH_3$ or phenyl

wherein the group(s) designated $[(R_1)_{n'}-Ar-(X)_b]_a$ is (are) attached directly to Ar.

2. The therapeutic composition according to Claim 1 wherein Ar is phenyl and $Ar_1$ is phenyl, quinolyl or indolyl.

3. The therapeutic composition according to Claims 1-2, wherein X is $-O(CHR_7)-$.

4. The therapeutic composition according to Claims 1-3 wherein "n" is 1.

5. The therapeutic composition according to Claims 1-4 wherein "b" is 1.

6. The therapeutic composition according to Claims 1-5 wherein "a" is 1.

7. A method for the treatment of hypersensitive, inflammatory or allergic conditions comprising the administration of a therapeutically effective amount of a compound according to Claims 1-6.

8. 4-p-Benzyloxyphenyl-3-buten-2-ol;
4-(2,4-Dibenzyloxyphenyl)-3-buten-2-ol;
1-(2,4-Dibenzyloxyphenyl)-1-octen-3-ol;
1-(2,4-Dibenzyloxyphenyl)-4,4-dimethyl-1-penten-3-ol;
1-(p-Benzyloxyphenyl)-5-N,N-dimethylamino-1-penten-3-one;
2',4'-Dibenzyloxycinnamyl alcohol;
4-(3,4-Dibenzyloxyphenyl)-3-but-en-2-ol;
4(Dipheny)methoxyphenyl)-3-buten-2-ol; or
4-(2,5-Dibenzyloxyphenyl)-3-buten-2-ol.